(19)

Europäisches
Patentamt
European
Patent Office
Office européen
des brevets

(11) **EP 4 715 059 A1**

(12) **EUROPEAN PATENT APPLICATION**
published in accordance with Art. 153(4) EPC

(43) Date of publication:
    **25.03.2026 Bulletin 2026/13**

(21) Application number: **24811054.6**

(22) Date of filing: **17.05.2024**

(51) International Patent Classification (IPC):
    *C12P 19/18* (2006.01)      *A23L 27/00* (2016.01)
    *A23L 33/125* (2016.01)     *A61K 8/60* (2006.01)
    *A61K 47/26* (2006.01)      *C07H 15/256* (2006.01)
    *C12N 9/10* (2006.01)       *C12N 9/24* (2006.01)
    *C12N 9/26* (2006.01)       *C12P 19/22* (2006.01)
    *C12P 19/50* (2006.01)

(52) Cooperative Patent Classification (CPC):
    A23L 27/00; A23L 33/125; A61K 8/60; A61K 47/26;
    C07H 15/256; C12N 9/10; C12N 9/24;
    C12N 9/2408; C12P 19/18; C12P 19/22;
    C12P 19/50

(86) International application number:
    **PCT/JP2024/018314**

(87) International publication number:
    **WO 2024/242047 (28.11.2024 Gazette 2024/48)**

(84) Designated Contracting States:
    **AL AT BE BG CH CY CZ DE DK EE ES FI FR GB
    GR HR HU IE IS IT LI LT LU LV MC ME MK MT NL
    NO PL PT RO RS SE SI SK SM TR**
    Designated Extension States:
    **BA**
    Designated Validation States:
    **GE KH MA MD TN**

(30) Priority: **19.05.2023 JP 2023083304**

(71) Applicant: **Amano Enzyme Inc.**
    **Nagoya-shi**
    **Aichi 460-8630 (JP)**

(72) Inventors:
    • **HAYASHI, Takuma**
      **Kakamigahara-shi, Gifu 509-0109 (JP)**
    • **KURITANI, Yuko**
      **Kakamigahara-shi, Gifu 509-0109 (JP)**

(74) Representative: **Mullholland, Lewis Paul**
    **WP Thompson**
    **44 Southampton Buildings**
    **London WC2A 1AP (GB)**

(54) **METHOD FOR PRODUCING GLYCOSYLATED STEVIOL GLYCOSIDE COMPOSITION**

(57)     [Summary]
[Problems] To provide a novel technique for improving the taste of a glycosylated steviol glycoside composition.
[Solution] The present technique provides a method for producing a glycosylated steviol glycoside composition and a method for improving the taste of a glycosylated steviol glycoside composition, which include a step of treating a stevia extract and dextrin with cyclodextrin glucanotransferase derived from a microorganism belonging to the genus Anoxybacillus. The production method and taste improvement method according to the present technique may further include a step of treating with a carbohydrate-processing enzyme other than the cyclodextrin glucanotransferase derived from a microorganism belonging to the genus Anoxybacillus.

EP 4 715 059 A1

**Description**

Technical Field

The present technique relates to a method for producing a glycosylated steviol glycoside composition. More specifically, the present technique relates to a method for producing a glycosylated steviol glycoside composition, a method for improving the taste of a glycosylated steviol glycoside composition, a taste improver for a glycosylated steviol glycoside composition, a glycosylated steviol glycoside composition, and a method for producing a taste-improved glycosylated steviol glycoside composition.

Background Art

In recent years, there has been an increasing demand for low-calorie sweeteners due to health-conscious trends and dieting, and steviol glycosides extracted and isolated from the leaves of the stevia plant have attracted attention as natural sweeteners. Stevia plants contain stevioside as the major steviol glycoside component, along with small amounts of rebaudioside A and trace amounts of other steviol glycosides. Although stevioside is about 100 to 300 times sweeter than sucrose, it has a bitter and astringent taste, raising concerns that it does not have a taste comparable to that of sucrose.

Under these circumstances, various techniques have been investigated to improve the taste of a stevia extract, such as adding glucose to a stevia extract. For example, Patent Literature 1 discloses a technique for reducing unpleasant odors, bitterness, long-lasting aftertaste, etc., by producing a glycosylated steviol glycoside composition in the presence of a glucose donor using cyclodextrin glucanotransferase derived from Thermoanaerobacter.

Furthermore, Patent Literature 2 discloses a technique for producing a highsweet sugar-added stevia sweetener having a good taste, by treating a stevia extract and dextrin with cyclodextrin glucanotransferase in the presence of $\gamma$-cyclodextrin to produce an $\alpha$-glucosylated stevia extract, and further treating the resulting an $\alpha$-glucosylated stevia extract with soybean-derived $\beta$-amylase.

Citation List

Patent Literature

[0005]

　　Patent Literature 1: Translation of PCT Application Publication No. 2019-517823
　　Patent Literature 2: Japanese Patent Application Laid-Open No. 3-83558

Summary of Invention

Technical Problem

As mentioned above, various techniques for improving the taste of glycosylated steviol glycoside compositions are being developed, but problems such as bitterness and a long-lasting aftertaste still remain, and further improvements are required.

Therefore, the main object of the present technique is to provide a novel technique for improving the taste of glycosylated steviol glycoside compositions.

Solution to Problem

As a result of intensive research to solve the above-mentioned problems, the inventors of the present application have unexpectedly discovered that the taste-improving effect varies depending on the origin of the enzyme used in the production of glycosylated steviol glycosides. Then, the origin of the enzyme that has great effect in improving taste has been identified, leading to completion of the present technique.

That is, the present technique provides a method for producing a glycosylated steviol glycoside composition and a method for improving the taste of a glycosylated steviol glycoside composition, which include a step of treating a stevia extract and dextrin with cyclodextrin glucanotransferase derived from a microorganism belonging to the genus Anoxybacillus.

The production method and taste improvement method according to the present technique can further include a step of treating with a carbohydrate-processing enzyme other than the cyclodextrin glucanotransferase derived from a microorganism belonging to the genus Anoxybacillus.

In this case, the carbohydrate-processing enzyme that can be used is one or more carbohydrate-processing enzymes selected from β-amylase, α-amylase, maltotriose synthase, transglucosidase, and maltotriosyltransferase.

Next, the present technique provides a taste improver for a glycosylated steviol glycoside composition, which comprises cyclodextrin glucanotransferase derived from a microorganism belonging to the genus Anoxybacillus.

The taste improver according to the present technique may further contain a carbohydrate-processing enzyme other than the cyclodextrin glucanotransferase derived from a microorganism belonging to the genus Anoxybacillus.

In this case, the carbohydrate-processing enzyme that can be used is one or more carbohydrate-processing enzymes selected from β-amylase, α-amylase, maltotriose synthase, transglucosidase, and maltotriosyltransferase.

The present technique also provides a glycosylated steviol glycoside composition in which the taste improver according to the present technique is used.

The present technique further provides a method for producing a taste-improved glycosylated steviol glycoside composition, which includes a step of treating a glycosylated steviol glycoside composition with a carbohydrate-processing enzyme other than cyclodextrin glucanotransferase derived from a microorganism belonging to the genus Anoxybacillus.

In the method for producing a taste-improved glycosylated steviol glycoside composition according to the present technique, one or more carbohydrate-processing enzymes selected from β-amylase, α-amylase, maltotriose synthase, transglucosidase, and maltotriosyltransferase can be used as the carbohydrate-processing enzyme.

Description of Embodiments

Preferred embodiments for carrying out the present technique will be described below. Note that the embodiments described below are examples of typical embodiments of the present technique, and the scope of the present technique should not be construed as being narrowed by those embodiments.

1. Method for producing glycosylated steviol glycoside composition and method for improving taste

The method for producing a glycosylated steviol glycoside composition and the method for improving the taste of a glycosylated steviol glycoside composition according to the present technique include a step of treating a stevia extract and dextrin with cyclodextrin glucanotransferase (hereinafter also referred to as "CGTase") derived from a microorganism belonging to the genus Anoxybacillus (hereinafter also referred to as the "CGTase treatment step"). If necessary, a step of treating with a carbohydrate-processing enzyme other than the cyclodextrin glucanotransferase derived from a microorganism belonging to the genus Anoxybacillus (hereinafter also referred to as the "carbohydrate-processing enzyme treatment step"), a recovery step, etc., may also be performed. The raw materials and enzymes used in the method for producing a glycosylated steviol glycoside composition and the method for improving the taste of a glycosylated steviol glycoside composition according to the present technique, as well as each step, are described in detail below.

(1) Raw materials

In the method for producing a glycosylated steviol glycoside composition and the method for improving the taste of a glycosylated steviol glycoside composition according to the present technique, a stevia extract and dextrin are used as raw materials.

[Stevia extract]

The stevia extract used in the present technique is an extract of steviol glycosides derived from a stevia plant. The variety of the stevia plant is not particularly limited, and may be either a natural variety and/or a hybrid. The part of the plant from which the stevia is extracted is also not particularly limited, and examples include roots, stems, leaves, and flowers, and in the present technique, an extract derived from the leaves of a stevia plant is preferred.

Steviol glycosides contained in a stevia extract refer to steviol glycoside compounds (steviol glycosides) found in stevia plants, such as rebaudioside A, rebaudioside B, rebaudioside C, rebaudioside D, rebaudioside E, rebaudioside F, rebaudioside M (also known as "rebaudioside X"), rubusoside, dulcoside, stevioside, etc. In naturally occurring steviol glycosides, the steviol glucose units are linked in the beta configuration.

The content of steviol glycoside compounds contained in a stevia extract is not particularly limited, and examples thereof include an extract composed of 28-30% stevioside, 50-55% rebaudioside A, 9-12% rebaudioside C, 1-3% rebaudioside F, and other steviol glycoside compounds, on a dry weight basis, with a total steviol glycoside content of 90% or more, preferably 95% or more; an extract composed of 25-30% stevioside, 55-65% rebaudioside A, and other steviol glycoside compounds , with a total steviol glycoside content of 95% or more; an extract composed of 55-65% rebaudioside A, 20-30% stevioside, and 3-8% rebaudioside C and dulcoside A ,with a total steviol glycoside content of 90% or more; and an extract in which multiple purified steviol glycoside compounds are mixed in any ratio.

The form of a stevia extract that can be used in the present technique is not particularly limited as long as it does not impair the action and effect of the present technique, but it is preferable to use a stevia extract formulated as a liquid or powder.

[Dextrin]

In this technique, dextrin is used as the glucose donor. Dextrin is a polysaccharide obtained by hydrolysis of starch, and has a structure in which α-glucose is polymerized via α-(1→4) or α-(1→6) glycosidic bonds. Generally, a dextrose equivalent (DE) of approximately 10 to 20 is called maltodextrin, and a DE of 10 or less is called dextrin, and in this technique, dextrins including maltodextrin can be used as the glucose donor.

Dextrin, together with a stevia extract, is treated with cyclodextrin glucanotransferase derived from a microorganism belonging to the genus Anoxybacillus, which will be described later, to cleave one or more glucose units, which are then transferred to steviol glycoside compounds in the stevia extract.

The origin of the dextrin is not particularly limited, and one type or a combination of two or more types of dextrins obtained by hydrolyzing starch obtained from wheat, corn, potato, cassava (tapioca), sago, etc. can be used.

(2) Cyclodextrin glucanotransferase derived from microorganisms belonging to the genus Anoxybacillus

In this technique, by treating a stevia extract and dextrin with cyclodextrin glucanotransferase derived from a microorganism belonging to the genus Anoxybacillus, it is possible to obtain a glycosylated steviol glycoside composition with improved taste compared to glycosylated steviol glycoside compositions produced using cyclodextrin glucanotransferase derived from a microorganism belonging to other genera.

In this technique, "improved taste" means that undesirable taste of sweeteners, such as bitterness, astringency, and a long-lasting aftertaste, are reduced, and a good sweetness is achieved. "Good sweetness" means that the body, depth, mellowness, and mildness of the sweetness are close to those of ordinary sugar.

Examples of the microorganism belonging to the genus Anoxybacillus include Anoxybacillus caldiproteolyticus, Anoxybacillus pushchinoensis, Anoxybacillus flavithermus, and the like. In this technique, it is preferable to use cyclodextrin glucanotransferase derived from Anoxybacillus caldiproteolyticus (including also the one formerly known as Geobacillus stearothermophilus).

Cyclodextrin glucanotransferase (EC 2.4.1.19) is an enzyme involved in the formation of sugar chains, and is an enzyme that catalyzes the reaction of transferring a glucose unit necessary for the formation of a sugar chain to another sugar chain, and has hydrolysis and cyclodextrin-producing actions.

In a preferred embodiment, the enzyme has the activity of producing cyclodextrin with a degree of polymerization of 6 to 8 using a polymer composed of α-1,4-linked glucose, such as starch, as a substrate. The cyclodextrin glucanotransferase that can be used in the present technique may also be an enzyme that has other actions as long as it has the activity of producing cyclodextrin.

Here, "cyclodextrin glucanotransferase derived from a microorganism belonging to the genus Anoxybacillus" means a cyclodextrin glucanotransferase produced by a microorganism classified into the genus Anoxybacillus (whether a wild-type strain or a mutant strain), or a cyclodextrin glucanotransferase obtained by genetic engineering techniques using a cyclodextrin glucanotransferase gene. Therefore, a recombinant produced by a host microorganism into which a cyclodextrin glucanotransferase gene obtained from a microorganism belonging to the genus Anoxybacillus (or a gene obtained by modifying said gene) has been introduced also falls under the category of "cyclodextrin glucanotransferase derived from a microorganism belonging to the genus Anoxybacillus."

The cyclodextrin glucanotransferase used in the present technique can be prepared from a culture solution of a microorganism belonging to the genus Anoxybacillus. Specific preparation methods include methods of recovering cyclodextrin glucanotransferase from the culture solution or microbial bodies of a microorganism belonging to the genus Anoxybacillus. For example, when a cyclodextrin glucanotransferase-secreting microorganism is used, the microbial bodies can be recovered from the culture solution in advance by filtration, centrifugation, or the like, as necessary, and the enzyme can then be separated and/or purified. In contrast, when a cyclodextrin glucanotransferase-nonsecreting microorganism is used, the microbial bodies can be recovered from the culture solution in advance, if necessary, and then the microbial bodies can be disrupted by pressure treatment, ultrasonic treatment, or the like to extract the enzyme, which can then be separated and/or purified. The enzyme separation and/or purification method can be any known protein separation and/or purification method, without any particular limitation. Examples of such methods include centrifugation, UF concentration, salting out, and various chromatography methods using ion exchange resins, etc. The separated and/or purified enzyme can be powdered by a drying method such as freeze-drying or vacuum drying, or can be powdered using an appropriate excipient and/or drying aid in the drying method. The separated and/or purified enzyme can also be formulated as a liquid by adding an appropriate additive and sterilizing by filtration. In this technique, commercially available cyclodextrin glucanotransferase or cyclodextrin glucanotransferase preparations can also be used. Examples of a commercially available cyclodextrin glucanotransferase or cyclodextrin glucanotransferase preparations include cyclodextrin glucanotransferases derived from Anoxybacillus caldiproteolyticus available from Amano Enzyme Inc.

In the present technique, the amount of cyclodextrin glucanotransferase derived from a microorganism belonging to the genus Anoxybacillus used is not particularly limited as long as it does not impair the action and effect of the present technique. The lower limit of the amount of cyclodextrin glucanotransferase derived from a microorganism belonging to the genus Anoxybacillus used is, for example, 0.4 U or more, preferably 2 U or more, more preferably 4 U or more, even more preferably 6 U or more, and even more preferably 9 U or more per 1 gram of a stevia extract. By setting the lower limit of the amount of cyclodextrin glucanotransferase derived from a microorganism belonging to the genus Anoxybacillus used within this range, the taste improving effect can be further improved.

The upper limit of the amount of cyclodextrin glucanotransferase derived from a microorganism belonging to the genus Anoxybacillus used is, for example, 600 U or less, preferably 400 U or less, more preferably 120 U or less, even more preferably 20 U or less, still more preferably 15 U or less, and particularly preferably 11 U or less, per 1 gram of a stevia extract.

In the present technique, the cyclodextrin glucanotransferase activity is a value measured by the cyclodextrin glucanotransferase activity measurement method described in the Examples below.

(3) A carbohydrate-processing enzyme other than cyclodextrin glucanotransferase derived from microorganisms belonging to the genus Anoxybacillus

In this technique, the taste improvement effect can be further improved by using a carbohydrate-processing enzyme other than cyclodextrin glucanotransferase derived from microorganisms belonging to the genus Anoxybacillus in addition to cyclodextrin glucanotransferase derived from microorganisms belonging to the genus Anoxybacillus. Specifically, by using a carbohydrate-processing enzyme other than cyclodextrin glucanotransferase derived from microorganisms belonging to the genus Anoxybacillus, it is possible to significantly reduce undesirable taste of sweeteners, such as bitterness, astringency, and a long-lasting aftertaste, while maintaining the favorable sweetness of the glycosylated steviol glycoside composition.

As the carbohydrate-processing enzyme other than cyclodextrin glucanotransferase derived from microorganisms belonging to the genus Anoxybacillus (hereinafter simply referred to as "carbohydrate-processing enzyme") that can be used in the present technique, one or more carbohydrate-processing enzymes that can be used in the production of glycosylated steviol glycoside compositions can be freely selected and used, as long as the action and effect of the present technique are not impaired. Examples of carbohydrate-processing enzymes include $\beta$-amylase, $\alpha$-amylase, maltotriose synthase, transglucosidase, and maltotriosyltransferase. Among these, $\beta$-amylase is particularly preferably used in the present technique.

$\beta$-amylase (EC 3.2.1.2) is an exo-type enzyme that sequentially degrades $\alpha$-1,4 glycosidic bonds from the non-reducing end of starch in maltose (malt sugar) units. The type and origin of $\beta$-amylase that can be used in the present technique are not particularly limited. Examples thereof include $\beta$-amylases derived from plants such as wheat, barley, and soybean, and $\beta$-amylases derived from microorganisms such as the genus Bacillus (e.g., Bacillus flexus, Bacillus megaterium, Bacillus polymyxa, and Bacillus circulans); Streptomyces sp.; and Pseudomonas sp. These $\beta$-amylases can be used alone or in combination of two or more.

In the present technique, from the viewpoint of further improving the taste, it is preferable to use $\beta$-amylase derived from a microorganism, it is more preferable to use $\beta$-amylase derived from the genus Bacillus, and it is even more preferable to use $\beta$-amylase derived from Bacillus flexus.

The $\beta$-amylase can be prepared from the culture solution of the plant cells or microorganisms from which the $\beta$-amylase is derived. Specific preparation methods are similar to the above-mentioned method for preparing cyclodextrin glucanotransferase. This technique is not limited to natural (wild-type) $\beta$-amylase, and recombinant $\beta$-amylase can also be used. Commercially available $\beta$-amylase or $\beta$-amylase preparations can also be used. An example of a commercially available $\beta$-amylase or $\beta$-amylase preparation is $\beta$-amylase derived from Bacillus flexus available from Amano Enzyme Inc.

The $\beta$-amylase used in the present technique can be prepared from the culture solution of the microorganism or plant from which the $\beta$-amylase is derived. Specific preparation methods include methods of recovering $\beta$-amylase from the culture solution or microbial bodies of the microorganism or plant. For example, when a $\beta$-amylasesecreting microorganism is used, the microbial bodies can be recovered from the culture solution in advance by filtration, centrifugation, or the like, as necessary, and the enzyme can then be separated and/or purified. In contrast, when a $\beta$-amylasenonsecreting microorganism or plant is used, the microbial bodies and plants can be recovered from the culture solution in advance, if necessary, and then the microbial bodies and plants can be disrupted by pressure treatment, ultrasonic treatment, or the like to extract the enzyme, which can then be separated and/or purified. The enzyme separation and/or purification method can be any known protein separation and/or purification method, without any particular limitation. Examples of such methods include centrifugation, UF concentration, salting out, and various chromatography methods using ion exchange resins, etc. The separated and/or purified enzyme can be powdered by a drying method such as freeze-drying or vacuum drying, or can be powdered using an appropriate excipient and/or drying aid in the drying method. The separated and/or purified enzyme can also be formulated as a liquid by adding an appropriate additive and sterilizing by filtration.

In the present technique, the amount of β-amylase used is not particularly limited as long as it does not impair the action and effect of the present technique. The lower limit of the amount of β-amylase used is, for example, 0.3 U or more, preferably 1.5 U or more, more preferably 3 U or more, even more preferably 30 U or more, and even more preferably 60 U or more per 1 gram of a stevia extract. By setting the lower limit of the amount of β-amylase used within this range, the taste improving effect can be further improved.

The upper limit of the amount of β-amylase used is, for example, 2000 U or less, preferably 400 U or less, more preferably 200 U or less, even more preferably 150 U or less, still more preferably 100 U or less, and particularly preferably 70 U or less per 1 gram of a stevia extract.

Furthermore, the β-amylase activity per 1 U of the activity of cyclodextrin glucanotransferase derived from a microorganism belonging to the genus Anoxybacillus is not particularly limited as long as it does not impair the action and effect of the present technique. Examples of the lower limits of β-amylase activity per 1 U of cyclodextrin glucanotransferase activity include 0.07 U or more, 0.1 U or more, 0.5 U or more, 0.7 U or more, 1.0 U or more, 3.0 U or more, and 5.0 U or more. Examples of the upper limits of β-amylase activity per 1 U of cyclodextrin glucanotransferase activity include 700 U or less, 650 U or less, 500 U or less, 400 U or less, 200 U or less, 100 U or less, 80 U or less, 50 U or less, 30 U or less, and 10 U or less.

In the present technique, the β-amylase activity is a value measured by the β-amylase activity measurement method described in the Examples below, and one unit (1 U) is defined as the amount of enzyme that causes an increase in reducing power equivalent to 1 mg of glucose per minute using a potato starch solution (pH 5.0) as a substrate.

(4) CGTase treatment step

The CGTase treatment step is a step of treating a stevia extract and dextrin with cyclodextrin glucanotransferase derived from a microorganism belonging to the genus Anoxybacillus.

Various conditions in the treatment step can be freely set as long as they do not impair the effects of the present technique. For example, the pH, temperature, treatment time, etc. can be set depending on the physicochemical properties of the cyclodextrin glucanotransferase used, such as the optimal pH, stable pH range, optimal temperature, and temperature stability. The pH can be set, for example, to 3.0 to 11.0, preferably 4.0 to 10.0, more preferably 5.0 to 9.0, 5.0 to 8.0, or 5.0 to 7.0. The temperature can be set, for example, to 10°C to 95°C, preferably 20°C to 90°C, more preferably 30°C to 85°C, 40°C to 80°C, or 50°C to 75°C. The treatment time can be set, for example, to 10 minutes to 300 hours, 30 minutes to 250 hours, preferably 1 hour to 125 hours, more preferably 2 hours to 100 hours, 6 hours to 75 hours, or 12 hours to 50 hours. The optimal reaction conditions can be determined through preliminary experiments.

(5) Carbohydrate-processing enzyme treatment step

The carbohydrate-processing enzyme treatment step is a step of treating a stevia extract and dextrin, or a composition prepared via the CGTase treatment step, with a carbohydrate-processing enzyme other than cyclodextrin glucanotransferase derived from a microorganism belonging to the genus Anoxybacillus. The CGTase treatment step and the carbohydrate-processing enzyme treatment step can be performed simultaneously or in any order, but are preferably performed simultaneously or in the order of CGTase treatment step → carbohydrate-processing enzyme treatment step, and more preferably in the order of CGTase treatment step → carbohydrate-processing enzyme treatment step.

Various conditions in the carbohydrate-processing enzyme treatment step can be freely set as long as they do not impair the effects of the present technique. For example, the pH, temperature, treatment time, etc. can be set depending on the physicochemical properties of the carbohydrate-processing enzyme used, such as the optimal pH, stable pH range, optimal temperature, and temperature stability. When β-amylase is used as the carbohydrate-processing enzyme, the pH can be set, for example, to 3.0 to 10.0, preferably 5.0 to 8.0, and more preferably 5.5 to 7.0. The temperature can be set, for example, to 25°C to 80°C, preferably 30°C to 75°C, and more preferably 60°C to 70°C. The treatment time can be set, for example, to 10 minutes to 250 hours, preferably 1 hour to 100 hours, more preferably 2 hours to 50 hours, or 4 hours to 25 hours. Optimal reaction conditions can be determined through preliminary experiments.

(6) Recovery step

The recovery step is a step of recovering a glycosylated steviol glycoside composition produced through a CGTase treatment step and, if necessary, a carbohydrate-processing enzyme treatment step. Specific recovery methods can be one or a free combination of two or more general recovery methods used in the production of glycosylated steviol glycoside compositions, depending on the type of the glycosylated steviol glycoside composition to be produced.

The method for producing a glycosylated steviol glycoside composition and the method for improving the taste of a glycosylated steviol glycoside composition according to the present technique described above can be embodied as comprising the following steps (1) to (5).

(1) A step of preparing a stevia extract and dextrin
(2) A step of treating the prepared stevia extract and dextrin with cyclodextrin glucanotransferase derived from a microorganism belonging to the genus Anoxybacillus
(3) A step of treating the prepared stevia extract and dextrin or the composition prepared in step (2) with a carbohydrate-processing enzyme other than cyclodextrin glucanotransferase derived from a microorganism belonging to the genus Anoxybacillus
(4) A step of inactivating the enzyme
(5) A step of recovering the produced glycosylated steviol glycoside composition

Note that steps (3) to (5) are not essential and can be performed as needed. Steps (2) and (3) can be performed simultaneously. Furthermore, an enzyme inactivation step may be added after steps (2) and/or (3). The enzyme used in step (2) may be treated simultaneously with the enzyme used in step (3), or may be treated separately. When treated separately, the order of the treatments is not particularly limited. Further, when treated separately, an enzyme inactivation step may be added between each enzyme treatment as needed.

2. Taste improver for Glycosylated steviol glycoside composition

The taste improver for a glycosylated steviol glycoside composition according to the present technique contains cyclodextrin glucanotransferase derived from microorganisms belonging to the genus Anoxybacillus. If necessary, it may also contain carbohydrate-processing enzymes other than cyclodextrin glucanotransferase derived from microorganisms belonging to the genus Anoxybacillus and other components. Details of the cyclodextrin glucanotransferase derived from microorganisms belonging to the genus Anoxybacillus and the carbohydrate-processing enzymes other than cyclodextrin glucanotransferase derived from microorganisms belonging to the genus Anoxybacillus are the same as those of the cyclodextrin glucanotransferase derived from microorganisms belonging to the genus Anoxybacillus and the carbohydrate-processing enzymes used in the production method and taste improvement method described above, and therefore will not be described here.

(1) Other components

The taste improver for the glycosylated steviol glycoside composition according to the present technique can be used in combination with other components as long as the action and effect of the present technique are not impaired. Examples of other components that can be used include excipients, pH adjusters, colorants, flavoring agents, disintegrants, lubricants, stabilizers, enzymes, and other components commonly used in formulations. Furthermore, components with known or future functions can also be used in combination as appropriate depending on the purpose.

3. Glycosylated steviol glycoside composition

Glycosylated steviol glycoside compositions produced using the method for producing a glycosylated steviol glycoside composition of the present technique and the method for improving the taste of a glycosylated steviol glycoside composition of the present technique have better taste than glycosylated steviol glycoside compositions produced using cyclodextrin glucanotransferase, which is commonly used in the production of glycosylated steviol glycoside compositions. Specifically, the glycosylated steviol glycoside compositions of the present technique have less bitterness and astringency. Furthermore, the glycosylated steviol glycoside composition of the present technique is characterized by a good sweetness. The reason for this is presumably due to the high proportion of specific steviol glycosides in the glycosylated steviol glycoside composition, as shown in the Examples below.

The glycosylated steviol glycoside composition according to the present technique can be suitably used in any application requiring sweetness, such as foods and beverages, oral pharmaceuticals, intraoral pharmaceuticals, and oral care products (including pharmaceuticals and quasi-drugs) such as toothpaste and mouthwash.

Examples of foods and beverages include confectionery products (jelly candies, hard candies, gums, etc.), yogurt (full-fat dairy yogurt, low-fat dairy yogurt, non-dairy yogurt, lactose-free yogurt, and frozen versions thereof), dessert products such as ice cream, sweet bakery products (biscuits, cakes, rolls, pies, pastries, cookies, etc.), sweet bakery mixes for producing sweet bakery products, pie fillings (fruit pie fillings, nut pie fillings, etc.), cereal products (extruded cereals, flaked cereals, puffed cereals, etc.), cereal coating compositions, bread products (fermented bread, non-fermented bread, yeast bread, non-yeast bread (soda bread, etc.)), wheat flour-based breads, wheat flourfree breads (e.g., potato, rice, rye, etc.), gluten-free breads, bread mixes, frozen dairy products, meats, dairy products, condiments, snack bars (e.g., cereal, nut, seed, and/or fruit bars), soups, dressings, various mixes, ready-to-eat meals, baby foods, diet preparations, syrups, food coatings, dried fruits, sauces, gravies, spreads (e.g., jams/jelly, butter, etc.). The food and beverage products may also be animal feed products, such as pet food. The food and beverage products of the present technique may contain the glycosylated steviol glycoside composition as a coating or sugar coating formed on the surface of the food. Such coatings not only improve the flavor of the food but also extend the shelf life of the food.

Oral pharmaceuticals include, for example, lozenges, drinks, granules, powders, tablets, capsules, etc. Intraoral pharmaceuticals include, for example, sprays, ointments, creams, pastes, patches, etc. Oral care products include, for example, liquid toothpaste, toothpaste, mouthwash, breath fresheners, etc.

4. Method for producing taste-improved glycosylated steviol glycoside composition

The method for producing a taste-improved glycosylated steviol glycoside composition according to the present technique is a method comprising a step of treating a glycosylated steviol glycoside composition with a carbohydrate-processing enzyme other than cyclodextrin glucanotransferase derived from a microorganism belonging to the genus Anoxybacillus. That is, the method produces a glycosylated steviol glycoside composition with improved taste by treating an already produced glycosylated steviol glycoside composition with a carbohydrate-processing enzyme other than cyclodextrin glucanotransferase derived from a microorganism belonging to the genus Anoxybacillus. Details of the carbohydrate-processing enzyme other than cyclodextrin glucanotransferase derived from a microorganism belonging to the genus Anoxybacillus and the carbohydrate-processing enzyme treatment step are the same as those of the carbohydrate-processing enzyme and carbohydrate-processing enzyme treatment step used in the production method and taste-improving method described above, and therefore will not be described here.

EXAMPLES

The present technique will be described in more detail below based on examples. Note that the examples described below are examples of typical examples of the present technique, and the scope of the present technique should not be construed as being narrowed by those examples.

1. Raw materials

The raw materials and enzymes used in the examples are shown in Table 1 below.

[Table 1]

| Substance name | Product name | Distribution source |
|---|---|---|
| Stevia extract | Stevia-derived AVITAS 200 (stevia extract) | NitinouSeiken Co., Ltd. |
| Dextrin (cassava-derived) | Pinedex #2 | Matsutani Chemical Industry Co., Ltd. |
| Cyclodextrin glucanotransferase 1 | Anoxybacillus caldiproteolyticus-derived cyclodextrin glucanotransferase 1 | Amano Enzyme Inc. |
| Cyclodextrin glucanotransferase 2 | Cyclodextrin glucanotransferase 1 derived from microorganism belinging to genus Thermoanaerobacter | Novozymes |
| Carbohydrate-processing enzyme | Bacillus flexus-derived β-amylase | Amano Enzyme Inc. |

2. Enzyme activity measurement method

[Method for measuring cyclodextrin glucanotransferase activity]

Measurement was carried out in accordance with the method described in the Japan's Specifications and Standards for Food Additives (9th edition). The specific method is as follows.

20 mL of water was added to 1.0 g of potato starch, and 5 mL of sodium hydroxide reagent (1 mol/L) was gradually added while stirring to form a paste. After heating in a boiling water bath for 3 minutes with stirring, 25 mL of water was added, cooled under running water, and the pH was adjusted to 5.5 with acetic acid reagent (1 mol/L). Further water was added to make a 100 mL volume, which was used as the substrate solution. 10 mL of the substrate solution was measured and heated at 40°C for 10 minutes, followed by the addition and mixing of 1 mL of enzyme solution. After incubation at 40°C for 10 minutes, the reaction was stopped by the addition of 10 mL of hydrochloric acid reagent (0.1 mol/L). To 1 mL of this reaction solution, 10 mL of iodine-potassium iodide reagent (0.4 mmol/L) was added and mixed to prepare the test solution. The iodine-potassium iodide reagent was prepared by dissolving 10.0 g of potassium iodide and 1.0 g of iodine in water to make 100 mL, and then diluting 200 times with water. A comparison solution was prepared by using water instead of the reaction solution, and the absorbance at 660 nm of the test solution and the comparison solution was measured. The amount of enzyme that reduces the blue iodine coloration of starch by 1% in 1 minute was defined as 1 unit (U).

[Method for measuring activity of carbohydrate-processing enzyme (β-amylase)]

Measurement was carried out in accordance with the method described in the Japan's Specifications and Standards for Food Additives (9th edition). The specific method is as follows.

Potato starch was used as the substrate. It was pre-dried at 105°C for 2 hours. 1.0 g of the dried material thereof was weighed out, 20 mL of water was added, and 5 mL of sodium hydroxide reagent (2 mol/L) was gradually added with stirring to form a paste. The mixture was then heated in a water bath with stirring for 3 minutes, after which 25 mL of water was added. After cooling, the mixture was neutralized with hydrochloric acid reagent (2 mol/L) and hydrochloric acid reagent (0.1 mol/L). 10 mL of 1 mol/L acetic acid/sodium acetate buffer (pH 5.0) was added, and water was added to make a total of 100 mL to prepare the substrate solution. 10 mL of the substrate solution was weighed and heated at 37°C for 10 minutes. 1 mL of sample solution was added and immediately shaken. After heating at the same temperature for 10 or 30 minutes, 4 mL of Fehling's reagent was added and gently shaken. After heating in a water bath for 15 minutes, the mixture was cooled to 25°C or below. 2 mL of 30% potassium iodide solution and 2 mL of sulfuric acid (1→6) were added to prepare the test solution. Fehling's reagent was prepared immediately in use by mixing 1 volume of copper solution with 1 volume of alkaline tartrate solution, wherein the copper solution had been prepared by dissolving 34.66 g of fine copper(II) sulfate pentahydrate crystals in water to make 500 mL, and the alkaline tartrate solution had been prepared by weighing 173 g of sodium potassium (+)-tartrate tetrahydrate and dissolving 50 g of sodium hydroxide in water to make 500 mL. A separate comparison solution was prepared using 10 mL of water instead of the substrate solution, following the same procedure as for the test solution. The liberated iodine in the test solution and the comparison solution was titrated with 0.05 mol/L sodium thiosulfate solution. The endpoint was determined when 1-2 drops of soluble starch reagent were added as the titration approached the end point and the resulting blue color disappeared. The amount of enzyme that increases the reducing power equivalent to 1 mg of glucose per minute was defined as 1 unit (1 U), and this was calculated using the following formula:

$$\beta\text{-amylase activity (U/g, U/mL)} = \text{amount of glucose (mg)} \times 1/10 \times 1/M$$

$$\text{Amount of glucose (mg)} = (b - a) \times 1.6 \times f$$

a: Titration value of enzyme reaction solution (mL)
b: Titration value of blank solution (mL)
1.6: 1 mL of 0.05 mol/L sodium thiosulfate solution is equivalent to 1.6 mg of glucose
1/10: Unit conversion factor for reaction time (minutes)
M: Amount of sample in 1 mL of sample solution (g or mL)
f: Factor of 0.05 mol/L sodium thiosulfate solution (for quantitative analysis)

3. Experimental example

<Experimental Example 1>

In Experimental Example 1, the influence of difference in enzymes used in the production of glycosylated steviol glycoside compositions on glycosylated steviol glycoside compositions was investigated.

(1) Production of glycosylated steviol glycoside composition

[Example 1, Comparative Example 1]

6.0 g each of a stevia extract and dextrin were suspended in phosphate buffer (pH 5.5), and cyclodextrin glucanotransferase 1 or 2 was added. The suspension was incubated at 70°C for 24 or 48 hours. Cyclodextrin glucanotransferase 1 or 2 was used at 10 U per 1 g of a stevia extract. After the reaction, the mixture was heated in nearboiling water (approximately 95°C) for 30 minutes, and then the produced glycosylated steviol glycoside composition was allowed to cool to room temperature.

[Example 2, Comparative Example 2]

A glycosylated steviol glycoside composition was prepared in the same manner as in Example 1 and Comparative Example 1, and then β-amylase, an example of a carbohydrate-processing enzyme, was added and incubated at 60°C for 6 hours. The amount of β-amylase added was 65 U per 1.0 g of a stevia extract. After the reaction, the mixture was heated in nearly boiling water (approximately 95°C) for 30 minutes, and then the glycosylated steviol glycoside composition was allowed to cool to room temperature.

(2) Analysis of component composition of glycosylated steviol glycoside composition

The glycosylated steviol glycoside composition thus produced was analyzed using high performance liquid chromatography (HPLC) (Shimadzu Corporation: SPD-M20A) under the conditions shown in Table 2. Each component composition was calculated using the following mathematical formula (1).

Each component composition (%) = (each component peak area / total peak area) $\times$ 100 (1)           (1)

In this experimental example, the proportions of stevioside and the other two types of steviol glycosides in the produced glycosylated steviol glycoside composition were calculated.

[Table 2]

| Column | Shodex Asahipak NH2P-40 3E (3.0 mm I.D. x 250 mm) |
|---|---|
| Eluent | (A)CH$_3$CN:(B)H$_2$O |
| Column temperature | 50°C |
| Injection amount | 5$\mu$L |
| Analysis time | 60min |
| Detector | UV210nm |
| Flow rate | 0.35 mL/min |

(3) Sensory evaluation

The glycosylated steviol glycoside compositions thus produced were evaluated for bitterness and sweetness. Specifically, the glycosylated steviol glycoside compositions of each Example and Comparative Example were diluted with ionexchanged water at a ratio of 125 times by weight, and four panelists discussed and evaluated the bitterness and sweetness according to the following criteria. The evaluation was performed by spitting out the sample, rinsing the mouth at least four times before placing it in the mouth.

[Bitterness]

The bitterness was scored as 4 points, 3 points, 2 points, and 1 point, in order from weakest to strongest.

[Sweetness]

The sweetness was scored as 4 points, 3 points, 2 points, and 1 point, in order from best to worst.

(4) Results

The results are shown in Table 3 below.

[Table 3]

| | | Example 1 | Comparative Example 1 | Example 2 | Comparative Example 2 |
|---|---|---|---|---|---|
| Enzyme used | Anoxybacillus caldiproteolyticus-derived cyclodextrin glucanotransferase | 10U/g | - | 10U/g | - |
| | Cyclodextrin glucanotransferase derived from microorganism belonging to genus Thermoanaerobacter | | 10U/g | - | 10U/g |
| | Carbohydrate-processing enzyme, Bacillus flexus-derived β-amylase | - | - | 65U/g | 65U/g |
| Component composition | Stevioside | 8.5% | 9.8% | 8.7% | 14.9% |
| | Steviol glycoside 1 | 9.8% | 7.5% | 10.0% | 8.6% |
| | Steviol glycoside 2 | 3.2% | 2.6% | 7.8% | 4.0% |
| Sensory evaluation | Bitterness | 3 | 2 | 4 | 1 |
| | Sweetness | 4 | 4 | 2 | 1 |

(4) Discussion

As shown in Table 3, Example 1, which used cyclodextrin glucanotransferase derived from a microorganism belonging to the genus Anoxybacillus, showed a higher proportion of steviol glycoside 1 and steviol glycoside 2 than Comparative Example 1, which used cyclodextrin glucanotransferase derived from a microorganism belonging to the genus Thermoanaerobacter. Furthermore, evaluation of the bitterness of Example 1 and Comparative Example 1 showed that Example 1, which used cyclodextrin glucanotransferase derived from a microorganism belonging to the genus Anoxybacillus, showed less bitterness than Comparative Example 1, which used cyclodextrin glucanotransferase derived from a microorganism belonging to the genus Thermoanaerobacter.

A comparison between Example 2 and Comparative Example 2, which further used β-amylase as a carbohydrate-processing enzyme, revealed that Example 2, which used cyclodextrin glucanotransferase derived from a microorganism belonging to the genus Anoxybacillus, did not increase the proportion of the starting substrate stevioside, but did increase the proportions of steviol glycoside 1 and steviol glycoside 2, compared to Comparative Example 2, which used cyclodextrin glucanotransferase derived from a microorganism belonging to the genus Thermoanaerobacter. Furthermore, evaluation of the bitterness of Example 2 and Comparative Example 2 revealed that Example 2, which used cyclodextrin glucanotransferase derived from a microorganism belonging to the genus Anoxybacillus, was able to significantly reduce bitterness compared to Comparative Example 2, which used cyclodextrin glucanotransferase derived from a microorganism belonging to the genus Thermoanaerobacter. The sweetness of Example 2 and Comparative Example 2 tended to be lower than that of Example 1 and Comparative Example 1. However, Example 2, which used cyclodextrin glucanotransferase derived from a microorganism belonging to the genus Anoxybacillus, had a better sweetness than Comparative Example 2, which used cyclodextrin glucanotransferase derived from a microorganism belonging to the genus Thermoanaerobacter.

<Experimental Example 2>

In Experimental Example 2, the effects on glycosylated steviol glycoside compositions of using α-amylase, maltotriose synthase, transglucosidase, or maltotriosyltransferase as a carbohydrate-processing enzyme in addition to cyclodextrin glucanotransferase derived from a microorganism belonging to the genus Anoxybacillus in the production of glycosylated steviol glycoside compositions were investigated.

(1) Production of glycosylated steviol glycoside composition

Glycosylated steviol glycoside compositions were produced, by treating with α-amylase, maltotriose synthase, transglucosidase, or maltotriosyltransferase, instead of the β-amylase used in Example 2 of Experimental Example 1.

(2) Analysis of component composition of glycosylated steviol glycoside composition

Each of the glycosylated steviol glycoside compositions produced was analyzed using high performance liquid chromatography (HPLC).

(3) Results

The results of the component composition analysis confirmed that the proportions of steviol glycoside 1 and steviol glycoside 2 in each glycosylated steviol glycoside composition were increased. This suggests that, similar to the case where β-amylase was used, the bitterness was reduced and good sweetness was achieved.

**Claims**

1.    A method for producing a glycosylated steviol glycoside composition, comprising a step of treating a stevia extract and dextrin with cyclodextrin glucanotransferase derived from a microorganism belonging to the genus Anoxybacillus.

2.    The method for producing a glycosylated steviol glycoside composition according to claim 1, comprising a step of treating with a carbohydrate-processing enzyme other than the cyclodextrin glucanotransferase derived from a microorganism belonging to the genus Anoxybacillus.

3.    The method for producing a glycosylated steviol glycoside composition according to claim 2, wherein the carbohydrate-processing enzyme is one or more carbohydrate-processing enzymes selected from β-amylase, α-amylase, maltotriose synthase, transglucosidase, and maltotriosyltransferase.

4.    The method for producing a glycosylated steviol glycoside composition according to claim 3, wherein the carbohydrate-processing enzyme is β-amylase.

5.    A method for improving the taste of a glycosylated steviol glycoside composition, comprising a step of treating a stevia extract and dextrin with cyclodextrin glucanotransferase derived from a microorganism belonging to the genus Anoxybacillus.

6.    The method for improving the taste of a glycosylated steviol glycoside composition according to claim 5, comprising a step of treating with a carbohydrate-processing enzyme other than the cyclodextrin glucanotransferase derived from a microorganism belonging to the genus Anoxybacillus.

7.    The method for improving the taste of a glycosylated steviol glycoside composition according to claim 6, wherein the carbohydrate-processing enzyme is one or more carbohydrate-processing enzymes selected from β-amylase, α-amylase, maltotriose synthase, transglucosidase, and maltotriosyltransferase.

8.    The method for improving the taste of a glycosylated steviol glycoside composition according to claim 7, wherein the carbohydrate-processing enzyme is β-amylase.

9.    A taste improver for a glycosylated steviol glycoside composition, comprising cyclodextrin glucanotransferase derived from a microorganism belonging to the genus Anoxybacillus.

10.    The taste improver according to claim 9, comprising a carbohydrate-processing enzyme other than the cyclodextrin glucanotransferase derived from a microorganism belonging to the genus Anoxybacillus.

11.    The taste improver according to claim 10, wherein the carbohydrate-processing enzyme is one or more carbohydrate-processing enzymes selected from β-amylase, α-amylase, maltotriose synthase, transglucosidase, and maltotriosyltransferase.

12.    The taste improver according to claim 11, wherein the carbohydrate-processing enzyme is β-amylase.

13.    A glycosylated steviol glycoside composition in which the taste improver according to any one of claims 9 to 12 is used.

14.    A method for producing a taste-improved glycosylated steviol glycoside composition, comprising a step of treating a glycosylated steviol glycoside composition with a carbohydrate-processing enzyme other than cyclodextrin glucanotransferase derived from a microorganism belonging to the genus Anoxybacillus.

15. The method for producing a taste-improved glycosylated steviol glycoside composition according to claim 14, wherein the carbohydrate-processing enzyme is one or more carbohydrate-processing enzymes selected from β-amylase, α-amylase, maltotriose synthase, transglucosidase, and maltotriosyltransferase.

16. The method for producing a taste-improved glycosylated steviol glycoside composition according to claim 15, wherein the carbohydrate-processing enzyme is β-amylase.

# EP 4 715 059 A1

## INTERNATIONAL SEARCH REPORT

International application No.

**PCT/JP2024/018314**

### A. CLASSIFICATION OF SUBJECT MATTER

*C12P 19/18*(2006.01)i; *A23L 27/00*(2016.01)i; *A23L 33/125*(2016.01)i; *A61K 8/60*(2006.01)i; *A61K 47/26*(2006.01)i; *C07H 15/256*(2006.01)i; *C12N 9/10*(2006.01)i; *C12N 9/24*(2006.01)i; *C12N 9/26*(2006.01)i; *C12P 19/22*(2006.01)i; *C12P 19/50*(2006.01)i

FI: C12P19/18; C12P19/50; C12N9/10; C12N9/26 A; C12N9/24; A23L33/125; C12P19/22; A23L27/00 101A; C07H15/256; A61K47/26; A61K8/60

According to International Patent Classification (IPC) or to both national classification and IPC

### B. FIELDS SEARCHED

Minimum documentation searched (classification system followed by classification symbols)

C12P19/18; A23L27/00; A23L33/125; A61K8/60; A61K47/26; C07H15/256; C12N9/10; C12N9/24; C12N9/26; C12P19/22; C12P19/50

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| X | JP 2898688 B2 (NIPPON PAPER INDUSTRIES CO., LTD.) 02 June 1999 (1999-06-02) claim 1 | 1-16 |
| A | BINAY, Baris et al. A double mutant of highly purified Geobacillus stearothermophilus lactate dehydrogenase recognises L-mandelic acid as a substrate. Enzyme and Microbial Technology. 2013, vol. 52, pp. 393-399 abstract | 1-16 |
| A | JP 61-135589 A (HAYASHIBARA BIOCHEM LAB INC.) 23 June 1986 (1986-06-23) | 1-16 |

☐ Further documents are listed in the continuation of Box C.      ☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **10 July 2024** | **23 July 2024** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (July 2022)

## INTERNATIONAL SEARCH REPORT
### Information on patent family members

International application No.

**PCT/JP2024/018314**

| Patent document cited in search report | | | Publication date (day/month/year) | Patent family member(s) | | | Publication date (day/month/year) |
|---|---|---|---|---|---|---|---|
| JP | 2898688 | B2 | 02 June 1999 | (Family: none) | | | |
| JP | 61-135589 | A | 23 June 1986 | GB | 2169902 | A | |
| | | | | FR | 2574081 | A | |
| | | | | CA | 1335183 | A | |
| | | | | HK | 26593 | A | |
| | | | | SG | 121492 | G | |
| | | | | KR 10-1986-0005016 | | A | |
| | | | | SG | 121492 | A | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019517823 W

- JP 3083558 A